# EUROPEAN PATENT APPLICATION

(11) **EP 3 851 047 A1**
(43) Date of publication of application: **21.07.2021**
(21) Application number: 20216352.3
(22) Date of filing: 22.12.2020
(51) Int. Cl.: A61B 5/30, A61B 5/00, A61B 5/05, A61B 5/243

(54) **IMPROVED WIRELESS ELECTROCARDIOGRAPH**

(30) Priority: 23.12.2019 IT 201900025204
(71) Applicant: Praxe Srl, 60121 Ancona (IT)
(72) Inventor: FRANCO, ANTONIO, 60121 ANCONA (IT)
(74) Representative: Baldi, Claudio

(57) **Abstract**

Wireless electrocardiograph (100, 200, 300) comprising an integrated sensor (101, 201, 301) consisting of a receiving antenna able to receive electromagnetic signals generated by the variations of the cardiac electrical vector; a signal amplifier (102, 202, 302); a filtering unit (103, 203, 303); an A/D converter (104, 204, 304) and a wireless communication module (105, 205, 305).

## Description

The present invention relates to an optimized wireless electrocardiograph.

In particular, the present invention relates to a wireless electrocardiograph of the type used for diagnostic and monitoring activities, characterized by optimized operational features.

As it is known, an electrocardiograph is a diagnostic instrument able to record the electrical potentials related with the cardiac activity by means of external electrodes connected on the chest, on the wrists and on the ankles. It is one of the most popular electromedical instruments and it has been used for long for diagnostic and monitoring activities.

The potentials on the body surface are detected in some universally accepted positions in such a way to obtain comparable plots (derivations). The cardiac signal generates potential differences on the body surface that are detected with suitable electrodes. The electrical stimulation is originated from the breast node positioned in the right atrium in correspondence of the superior cava vena, and can be affected by several factors, causing a variation of the cardiac frequency. For instance, the frequency is increased by the sympathetic nervous system, whereas it is reduced by the parasympathetic nervous system. The transmission of the electrical stimulations produces electrical currents that are normally recorded with an electrocardiogram (ECG). In particular, three characteristic waveforms can be detected in a typical ECG signal: P wave (atrial depolarization), QRS complex (ventricular depolarization) and T wave (repolarization impulse). The duration, amplitude and morphology of said waveforms are clinically important because they can be directly correlated to the conditions of the conductive ways of the myocardium. The potentials on the cutaneous surface are not strictly correlated to the real cardiac potentials, but a good correlation degree can be obtained by representing said electrical activity as a product of the motion of a dipole with momentum characterized by variable parameters (intensity and direction). The potentials on the body surface are detected in universally accepted positions in such a way to obtain comparable plots (the so-called derivations). Every derivation corresponds to a characteristic recording track; the ECGs can simultaneously report and/or display one, three, six or twelve tracks, and the derivation to be recorded can be selected by means of a manual, electronic or automatic sequential switch. The electrical signal detected on the body surface has a relatively small amplitude (approximately 1 mV) and therefore the extent (the gain) and the quality of the amplification are especially important. The cardiac signal is detected by the electrodes, which are rigid or flexible metallic elements applied to the skin to detect the signal; they can be reusable or disposable electrodes. Reusable electrodes can be made of steel (plates, generally used in the derivations on arms and legs, or suction cups, used on the chest in the so-called precordial derivations), silver or silver chloride: although they are more expensive, the latter are more delicate, and ae mounted in self-adhesive supports that are usually very flexible in order to be fixed to the skin after shaving and cleaning. The electrodes are connected to the apparatus by means of the so-called patient cable, which is composed of a set of separate, very flexible electrical cables. The cardiac signal from the electrodes is amplified by a pre-amplifier provided at the input with a filter system that eliminates the continuous component and determines the minimum recordable frequency. The successive filtering system eliminates the noise components in as much as possible. The filtering circuits perform a selective attenuation of some specific frequency bands, in order to improve the readability of the plot and attenuate the interference signals. Practically speaking, all the electrocardiographs that are currently available on the market are configured to insert at least one filter able to attenuate the interference at the mains frequency without significantly altering the ECG signal. Some electrocardiograph models are suitably configured for the insertion of additional filters to reduce the effects of muscular tremor and the artifacts generated by the patient's movements and respiration. The signal detected by the electrodes is controlled with a failure detector and successively enters in the protection circuit in order to prevent an excessively high voltage, such as in the case of defibrillation, that can reach and damage the amplification circuits. Successively, a derivation selector is provided to suitably combine the signals from the electrodes connected to the patient so that the operator can select the derivations without changing the position of the electrodes. With reference to the patient, the derivations can be divided into derivations on the frontal plane and derivations on the transverse plane. The derivations on the frontal plane can be additionally divided into standard bipolar and unipolar derivations. In case of derivations on the frontal plane, the electrodes are positioned on the right arm (Right Arm, RA), left arm (Left Arm, LA), left leg (Left Leg, LL), whereas in the case of derivations on the transverse plane six electrodes are positioned in 90 degrees on the left side of the chest, and the RA-LA-LL mean voltage is used as potential reference. The calibrator allows the operator to manually introduce a signal with known constant amplitude by means of a suitable command in order to calibrate the instrument. The signal applied at the beginning of the amplification chain is typically 1 mV. The preamplifier is used to realize a first amplification of the ECG signal without distortion, and simultaneously minimize the interference and the artifacts. In some models, the pre-amplifier makes the signal available at its output in common mode and, after being suitably processed, the signal can be brought back to the input in order to additionally reduce the effect of the signal in common mode. An isolation circuit is provided after the pre-amplification circuit with a twofold function: firstly, to electrically insulate the patient from the electrical mains in order to prevent a potentially dangerous voltage from being applied to the patient by the electrodes in case of malfunctioning; secondly, to prevent dispersion currents of other devices eventually connected to the patient from affecting the measurement through the electrodes of the ECG and of its internal circuits towards the ground, creating a dangerous situation.

Isolation is basically obtained with two techniques: optical isolation and electromagnetic isolation. These techniques are often combined in the electrocardiographs that are commonly available on the market: optical isolation is preferred to transfer low-power signals (the ECG signal), whereas electromagnetic isolation is used to transfer a higher power (circuit power).

After a suitable filtering, the main amplifier is provided to amplify the electrocardiographic signal that has been processed in the preceding steps in such a way to drive the writing system. Generally, the circuits of this block are suitably configured to superimpose a continuous, variable component to the signal by means of a command, so that the operator can arrange the writing system on the baseline. The ADC is an A/D converter that, with an input analogue voltage, is able to provide an output multi-bit binary combination in a proportional way to the input. Then, such a bit combination is stored. The writing system or printer is the output component of the device. The function of the printer is to provide a graphic representation of the trend of the ECG signal over time. The printer includes a writing section and a paper dragging section. A first classification can be made between one-channel and multichannel recorders. One-channel recorders analyze the trend over time of the ECG signal relative to the various derivations in sequential mode, examining the track of one derivation at the time. Multi-channels recorders simultaneously analyze the signals that refer to multiple derivations. Currently, the most popular writing system is a printer with thermosensitive paper and galvanometer with heated pen; other systems use spray pens or cartridges on ordinary paper. The most recent systems tend to use thermal printers with high-resolution dot matrix. Some devices are provided with an LCD to display tracks and alphanumeric information.

Electrocardiograms can be performed at rest and in stress conditions to provide very useful information on the activity and the health of the heart, such as for example alterations of the cardiac rhythm, coronary disease, variations of the heart volume, and latent heart disease, in addition to measuring the resistance to physical activity, when performed under stress.

The electrical signal measured on the body surface has a relatively small amplitude (0.2-0.8 mV). However, the body surface does not only have the electrical potentials generated by the cardiac activity, but also disorders of different kind that are able to generate electrical potentials that are much bigger than the ones measured for the derivations. These disorders must be eliminated in as much as possible in order to obtain valid plots and noiseless derivations. In order to do so, both the continuous signals and the signals that oscillate with frequencies other than cardiac frequencies must be filtered. A large variety of professional electrocardiographs is currently available on the market, wherein software programs are implemented to accelerate the processing of the measurements that are still made with a traditional electric circuit composed of electrodes and connection cables. In particular, the potential interference sources and obstacles must be neutralized for a correct wireless measurement when they are proximal to the measurement device. Specifically, the main interference sources are: microwave ovens, 2.4 - 5 GHz cordless telephones, wireless speakers, cables with insufficient shielding, and other wireless devices.

The position of the electrodes significantly affects the type of measurement. In order to identify the ideal position of the electrodes, it is necessary to define the difference between linear conductor, which has the same voltage in all points of its length, and volume conductor, wherein the voltage can significantly vary in the different areas. For instance, the chest acts as a volume conductor and a precise positioning of the electrodes on the chest is fundamental for a correct recording. On the other hand, arms and legs act as linear conductors and can be therefore considered as extensions of the electrode cables.

Such a solution is described in the international patent application WO2016/145314A1, which describes a wireless ECG sensor system comprising a sensor patch configured to attach to a user. The sensor patch may comprise a substrate with a positive electrode and a negative electrode and a passive radio-frequency identification (RFID) transponder carried by the substrate. The RFID may comprise a first antenna, a non-transitory and nonvolatile storage medium in electrical communication with the first antenna, a load modulation switch in electrical communication with the first antenna, and a microcontroller in electrical communication with the first antenna and in data communication with both the storage medium and the load modulation switch. The system may also comprise an interrogator device comprising a second antenna configured to wirelessly transmit electromagnetic radiation having a resonant frequency of the first antenna of the sensor patch; and a demodulator configured to measure a voltage amplitude of the electromagnetic radiation wirelessly transmitted by the second antenna.

However, such a solution is impaired by the problem caused by the external interference, such as the interference due to electrostatic charges.

Another example is disclosed in the patent application US2013046195A1, which refers to an implantable medical device such as a pacemaker or a defibrillator, and comprises a programmable detection circuit that provides a signal that approximates an electrocardiogram (ECG) by means of implanted electrodes. With various configurations of electrodes, the signals that approximate various ECG signals are acquired without the need to connect electrodes with cables on the skin. Moreover, the various configurations of electrodes comprise combinations of electrodes of intracardiac stimulation, portions of the implantable medical device in contact with the tissue and electrodes embedded in the surface of the implantable medical device.

Another solution is provided in the patent application WO2012021488A1, which discloses an implantable medical device and a method for monitoring the changes in the transimpedance in a body tissue due to variations in the cardiac activity. A first dipole is used to deliver a non-stimulating electrical current, the first dipole comprising a first electrode and a second electrode adapted to be deployed along a first body location. A second dipole is used to measure a voltage signal resulting from the non-stimulating electrical current being conducted through a portion of a patient's body. The second dipole comprises a third electrode and a fourth electrode adapted to be deployed along a second body location spaced apart from the first body location.

An additional solution of the prior art is disclosed in the patent CN1012488991 A, which discloses a wireless device used to measure the cardiac electrical activity, said device comprising an electrode and a Bluetooth module. The device can transmit the ECG data to a cellular telephone via Bluetooth, said telephone transmitting the data to a medical assistance center. The device comprises an integrated circuit (Single Chip Module) connected with a data memory, a Bluetooth module and a display connected by means of the serial interfaces of the integrated circuit, a measurement circuit of the cardiac signal having an input terminal of the signal connected with the integrated circuit by means of a D/A converter of the chip, and an output terminal of the signal connected with the input terminal of the signal of an A/D converter of the chip. Moreover, the device comprises a first electrode and a second electrode, respectively connected to the input terminal of the signal of the measurement circuit of the cardiac signal, and a keyboard connected to an I/O interface of the chip.

In spite of being advantageous under different aspects, the major problem of the wireless electrocardiographs of the prior art consists in the fact that they are not immune to the electrical interference of other devices.

The purpose of the present invention is to disclose an optimized wireless electrocardiograph able to make measurements in a wireless mode and characterized by features able to overcome the limitations of the electrocardiographs of the prior art.

An optimized wireless electrocardiograph is disclosed according to the present invention, as claimed in claim 1.

For a better understanding of the present invention, a preferred embodiment of the invention will be described hereinafter for merely illustrative, not limiting purposes, with reference to the appended drawings, wherein:
- Fig. 1 is a diagrammatic view of a first embodiment of an optimized wireless electrocardiograph according to the invention;
- Fig. 2 is a diagrammatic view of a system of integrated sensors of an optimized wireless electrocardiograph according to the invention;
- Fig. 3 is a diagrammatic view of a second embodiment of an optimized wireless electrocardiograph according to the invention;
- Fig. 4 is a diagrammatic view of the system integrated with the second embodiment of an optimized wireless electrocardiograph according to the invention;
- Fig. 5 is a diagrammatic view of the system integrated with a third embodiment of an optimized wireless electrocardiograph according to the invention.

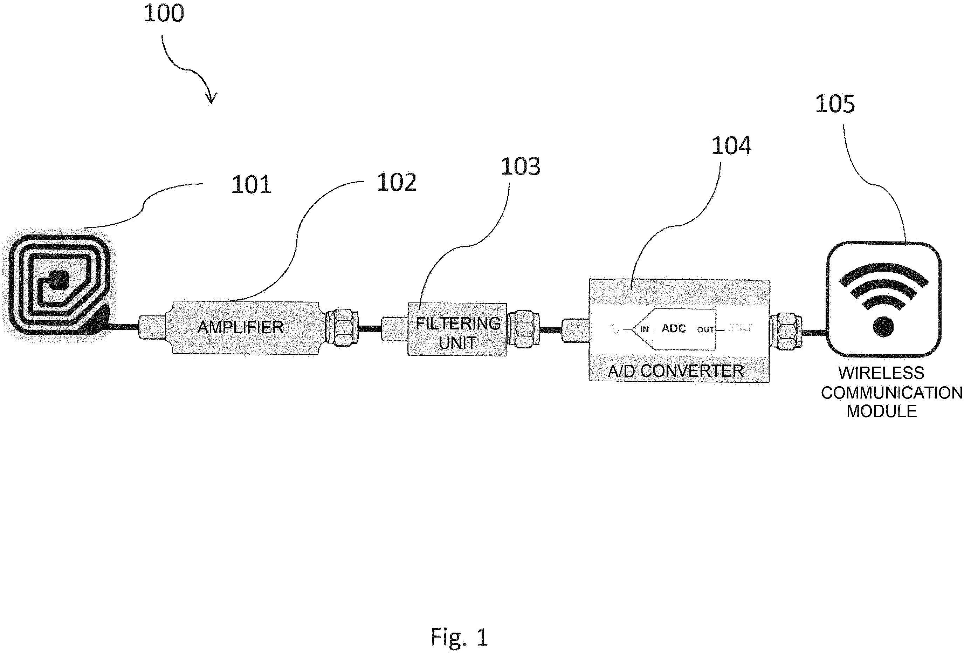

With reference to Figs. 1-5, an optimized wireless electrocardiograph 100, 200, 300 is disclosed according to the invention.

In particular, the wireless electrocardiograph 100, 200, 300 comprises an integrated sensor 101, 201, 301 consisting of a receiving antenna able to receive electromagnetic signals generated by the variations of the cardiac electrical vector; a signal amplifier 102, 202, 302; a filtering unit 103, 203, 303; an A/D converter 104, 204, 304 and a wireless communication module 105, 205, 305.

According to an aspect of the invention, the receiving antenna 101 is configured to be placed in contact with the body.

According to another aspect of the invention, the amplifier 102, 202, 302, the filtering unit 103, 203, 303, the A/D converter 104, 204, 304, and the wireless communication module 105, 205, 305 are miniaturized and housed into a single container.

According to an additional aspect of the invention, the filtering unit 103, 203, 303 can be a filter or a function executed by a management software.

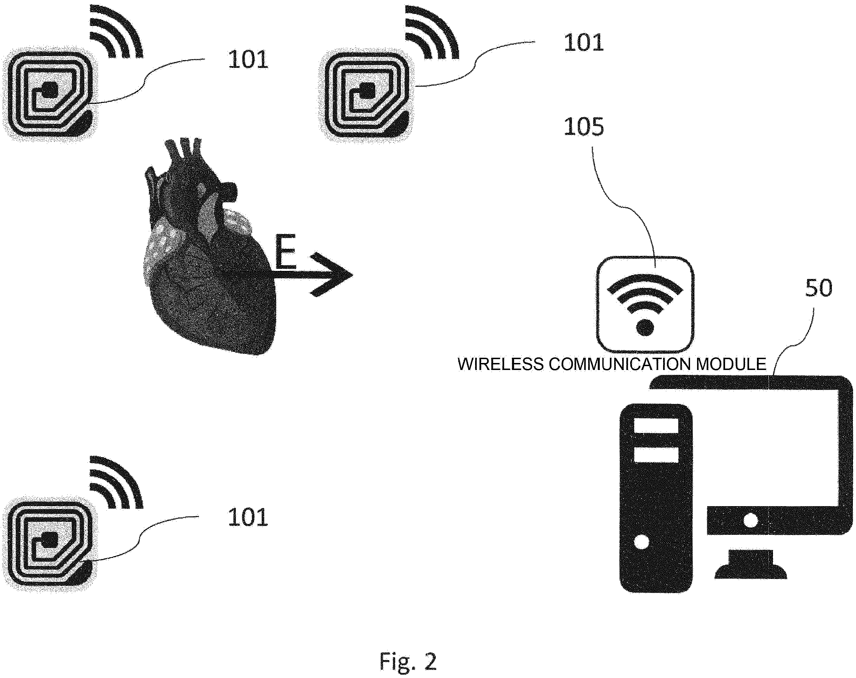

As shown in Fig. 2, a plurality of sensors 101 can be used to send a plurality of signals to a computer 50 able to analyze said signals by means of a software and to produce the derivations expected by the medical protocols.

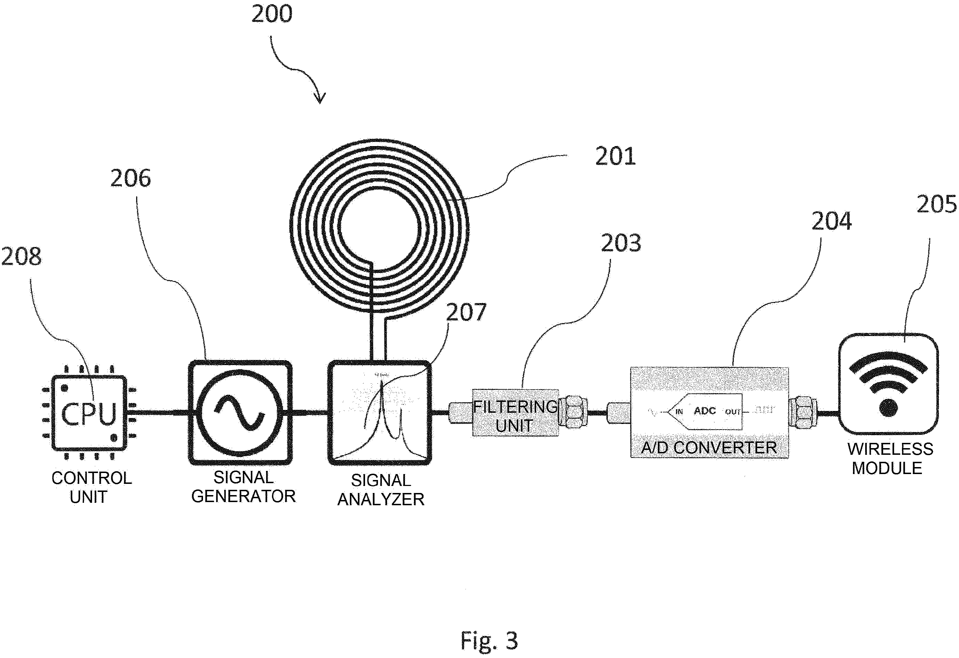

As shown in Fig. 3, according to a second embodiment, the wireless electrocardiograph 200 comprises an integrated sensor 201 consisting of a coil able to be crossed by an electrical current, a filtering unit 203, an A/D converter 204, and a wireless module 205. Useful data for monitoring the electric activity of the heart can be obtained from studying the interaction between the signals produced by the variations of the cardiac electric vector and the signals produced by the coil. In fact, the coil is configured to be placed in contact with the body.

According to an aspect of the invention, the wireless electrocardiograph 200 also comprises a signal generator 206, a signal analyzer 207, a control unit 208 to program specifically to perform the operations required to obtain the derivations.

The coil can be also used without using an external current to record induced currents directly. Currently, the electrical instruments of the prior art that use similar devices have a high sensitivity. The signals from one or more sensors 201 are sent to a computer able to analyze said signals by means of a software and to produce the derivations expected by the medical protocols.

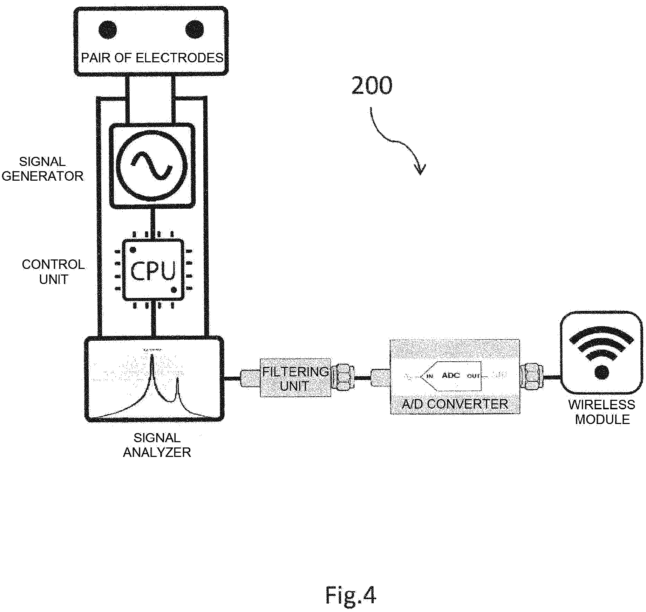

As shown in Fig. 4, a system according to the second embodiment of an optimized wireless electrocardiograph consists in a traditional system with traditional electrodes where a low-intensity alternate current is applied. The system comprises a pair of close electrodes to be placed in contact with the body of a user; also in this case, in addition to the sections dedicated to the signal generator and to the signal analyzer, a control unit is provided and programmed specifically to perform the operations required to obtain the derivations. The frequency is scanned, searching for the effects of the interference and resonance phenomena generated by the variations of the cardiac vector. According to this embodiment of the invention, the pairs of electrodes are positioned at a close distance to act as an integrated sensor, composed of the pair of electrodes, a variable frequency signal generator, a signal amplitude analyzer, an A/D converter and a wireless communication system. The system comprises a software to interpret the data and reconstruct the derivations. The applicant made some measurement tests to optimize the wireless electrocardiograph, using combined platinum potentiometric electrodes, composed of a plate or ring of Pt, a silver wire covered with AgCI, and a KCI solution.

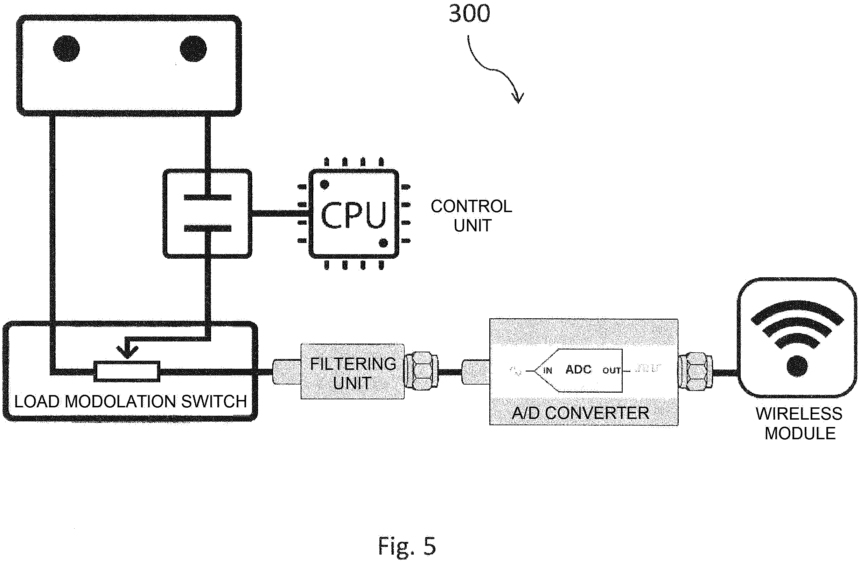

As shown in Fig. 5, according to a third embodiment of the invention, an integrated sensor consists of two electrodes, a variable capacitive element, a potentiometer, an A/D converter, a wireless communication system, and a control unit to program specifically to perform the operations required to obtain the derivations

The potential values that are measured, digitalized and wirelessly transmitted to the central receiver are successively processed by a suitable software to be specifically developed, because they cannot be processed by an echocardiograph of the prior art; therefore, a new 2.0 electrocardiograph technology will be developed to manage the digitalized wireless signals.

Finally, it must be noted that the signals from the sensors must be filtered in order to analyze the data from the cardiac vector for all the proposed cases. Such a process can be directly executed in the integrated sensor by inserting the additional components (low cut filters and high cut filters) or at analysis software level, with the advantages due to the higher processing capabilities of the signal and to the possibility of an upgrade. On the other side, the wireless transmission system would be loaded with a high data traffic. It is therefore necessary to compromise between the two options, based on the technologies that will be available when the devices are experimentally developed.

Therefore, the optimized wireless electrocardiograph according to the invention is able to avoid interference with other electrical devices.

Moreover, the optimized wireless electrocardiograph according to the invention comprises optimized sensors that are suitable for measuring the potentials.

Furthermore, the optimized wireless electrocardiograph according to the invention allows for using a software to process the signals that comprises frequency filters.

Additionally, the optimized wireless electrocardiograph according to the invention allows for integrating the modules in a compact apparatus.

Moreover, the optimized wireless electrocardiograph according to the invention is not invasive because of its compact size, is easy to handle and has a low emotional impact in pediatric patients.

Moreover, the optimized wireless electrocardiograph according to the invention is the ideal solution for rare diseases, such as the ones that are a cause of sudden death in patients, especially young ones, which imply a QT alteration. In fact, because of the reliability of the 12 derivations (instead of the 3 derivations of a Holter device) and because of its compact dimensions, the optimized wireless electrocardiograph can be worn by the patient for 24 hours and allows the doctor to detect intermittent alterations of the plot that are not necessarily visible during the standard tests carried out in a medical practice or with the instruments that are currently available for a 24-hour monitoring.

Moreover, the optimized wireless electrocardiograph according to the invention is the ideal solution for surgical operations whenever it is necessary to monitor the patient with an ECG, when the presence of cables may be a problem and may cause difficulties, requiring the execution of an ECG prior to the operation, without the possibility of monitoring the data during the operation.

Furthermore, the optimized wireless electrocardiograph according to the invention is the ideal solution for an intracavitary ECG (which is also used to assess the correct position of the central venous access point) wherein an intracavitary electrode is currently connected with a traditional cable to the needle of a syringe, and is moved towards the atrium, whereas two other electrodes are disposed on the surface in their usual positions.

Additionally, the optimized wireless electrocardiograph according to the invention is an innovative instrument suitable for being applied in preventive medicine, because the ECG that is currently performed in the sports medicine departments of public and private hospitals is one of the few mandatory general screening tests.

Modifications and variations can be made to the optimized wireless electrocardiograph according to the invention, without leaving the protection scope of the present invention, as claimed in the appended claims.

## Claims

1. Wireless electrocardiograph (100, 200, 300) comprising an integrated sensor (101, 201, 301) consisting of a receiving antenna able to receive electromagnetic signals generated by the variations of the cardiac electrical vector; a signal amplifier (102, 202, 302); a filtering unit (103, 203, 303); an A/D converter (104, 204, 304) and a wireless communication module (105, 205, 305).

2. Wireless electrocardiograph (100, 200, 300) according to claim 1, **characterized in that** the receiving antenna (101) is configured to be placed in contact with the body.

3. Wireless electrocardiograph (100, 200, 300) according to claim 1, **characterized in that** the signal amplifier (102, 202, 302), the filtering unit (103, 203, 303), the A/D converter (104, 204, 304), and the wireless communication module (105, 205, 305) are miniaturized and housed into a single container.

4. Wireless electrocardiograph (100, 200, 300) according to claim 1, **characterized in that** the filtering unit (103, 203, 303) is a filter or a function executed by a management software.

5. Wireless electrocardiograph (100, 200, 300) according to claim 1, **characterized in** comprising a plurality of sensors (101) used to send a plurality of signals to a computer (50) able to analyze said signals by means of a software and to produce the derivations expected by medical protocols.

6. Wireless electrocardiograph (100, 200, 300) according to claim 1, **characterized in** comprising an integrated sensor (201) consisting of a coil able to be crossed by an electrical current, a filtering unit (203), an A/D converter (204), a wireless communication module (205).

7. Wireless electrocardiograph (100, 200, 300) according to claim 1, **characterized in** comprising a signal generator (206), a signal analyzer (207), a control unit (208) to program specifically to perform the operations required to obtain the derivations.

8. Wireless electrocardiograph (100, 200, 300) according to claim 1, **characterized in** being integrated in a standard system having conventional electrodes to which a low-intensity alternate electrical current is applied, said system comprising a pair of close electrodes to be placed in contact with the body of a user.

9. Wireless electrocardiograph (100, 200, 300) according to claim 1, **characterized in that** the integrated sensor consists of two electrodes, a variable capacitive element, a potentiometer, an A/D converter, a wireless communication module, and a control unit to program specifically to perform the operations required to obtain the derivations.
